# EUROPEAN PATENT APPLICATION

(11) **EP 1 300 169 A1**
(43) Date of publication of application: **09.04.2003**
(21) Application number: 01830636.5
(22) Date of filing: 08.10.2001
(51) Int. Cl.: A61M 5/32

(54) **Blood sample collection apparatus with a simplified safety device**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

An apparatus (100) for collecting blood samples comprises a sleeve (1) having a substantially cylindrical body (10), hollow on the inside, defining an inner chamber (11) open at the front and rear, a needle assembly (5) comprising a needle-carrier (50) which carries at its opposite ends a first external needle (51) designed to be inserted into the patient's vein and a second internal needle (52) designed to engage in a vacutainer-type test tube, and a safety device (7) having a substantially cylindrical hollow body (70) defining an inner chamber (71) able to receive the vacutainer-type test tube. The safety device is able to support the needle assembly (5) so that the internal needle (52) is disposed axially inside the chamber (71) of the safety device and the safety device (7) is mounted movably inside the chamber (11) of the sleeve (1) to pass from a working position in which the external needle (51) protrudes axially outward from the sleeve (1) to a safe position in which the external needle (51) is protected inside the chamber (11) of the sleeve (1) and the internal needle (52) is protected inside the chamber (71) of the safety device (7).

## Description

The present invention refers to a blood sample collection apparatus with a simplified safety device.

An apparatus for collecting blood samples generally comprises a body or sleeve to which is fixed a needle-carrier that supports two needles, an external needle facing axially toward the outside of the body and an internal needle facing axially toward the inside of the body. The external needle is placed in the patient's vein and a vacutainer-type test tube, that is a test tube subjected to an internal vacuum, is positioned inside the sleeve and pierced by the internal needle. In this manner, thanks to the vacuum in the vacutainer, blood is drawn from the patient's vein through the needles into the test-tube, without the operator having to perform any manual operations.

Once the sample has been collected, the operator removes the test tube filled with blood to take it to a centre for analysis or collection and then sends the collection device including the sleeve and the needle for disposal. This operation is extremely dangerous for the operator who risks injury with the needle of the collection device. Moreover, the external needle of the collection device in any case remains exposed, with the resulting risk of injury for those responsible for disposal.

In order to avoid this drawback various safety devices for protection of the needle are known on the market.

Special caps which are placed irremovably on the head of the external needle are known to the art. This solution presents drawbacks due to difficulty in inserting said cap.

Safety devices comprising a sleeve slidingly mounted on the body of the collection device and movable from a retracted position in which it leaves the needle exposed to allow collection to an extended position in which it covers the needle are known to the art.

Said safety device presents the drawback of increasing the outside diameter of the sampling device, causing problems in insertion and correct positioning of the external needle in the patient's vein, since the needle proves to be excessively inclined with respect to the vein, with the risk of double puncture of the vein.

Blood sample collection devices are also known which have at the top of the sleeve a needle-carrier clamping mechanism which can be operated by the operator, by means of a pushbutton, to clamp/unclamp the needle-carrier. In this manner, on completion of sampling, the operator operates the clamping mechanism to cause expulsion of the needle into an appropriate container. Said solution is excessively complex and costly owing to the provision of the clamping mechanism and the separate container for collection of the needle.

The object of the present invention is to eliminate the drawbacks of the prior art, providing an apparatus for collection of blood samples that is able to ensure a high degree of safety against the possibility of accidental injury.

Another object of the present invention is to provide such an apparatus for collection of blood samples that is practical, versatile, structurally simple, economical and easy to make.

These objects are achieved according to the invention with the characteristics listed in appended independent claim 1.

Advantageous embodiments of the invention will become apparent from the dependent claims.

The blood sample collection apparatus according to the invention comprises a body or sleeve defining an inner chamber open at the front and rear, a needle assembly comprising a needle-carrier which bears at its opposite ends a first needle designed to be inserted in the vein of the patient and a second needle designed to engage in the vacutainer-type test tube.

The peculiar characteristic of the invention is represented by the fact that the needle assembly is mounted in the top of a safety device defining an inner chamber adapted to receive a vacutainer type test tube. The safety device is mounted movably within the sleeve to pass from a working position wherein the external needle protrudes axially form the top of the sleeve and a safe position in which the external needle is protected inside the sleeve.

The advantages of the apparatus for collection of blood according to the invention are evident. In fact, once the blood sample has been collected, the operator acts manually on the safety device to bring it into the safe position, in which the external needle is protected by the sleeve and the internal needle is protected by the safety device, thus avoiding accidental injury and needle stabs.

The apparatus for blood sample collection according to the invention proves economical and simple to make since it comprises, as an additional component, only the safety device which besides is simple in structure. Moreover said apparatus for blood collection does not increase the dimensions of the sleeve and does not require additional needle collection devices. In fact the safety device works inside the sleeve and the needle assembly is protected by exploiting the chambers of the sleeve and of the safety device.

Further characteristics of the invention will be made clearer by the detailed description that follows, referring to a purely exemplary and therefore non-limiting embodiment thereof, illustrated in the appended drawings, in which:
Figure 1 is an exploded axonometric view, illustrating the apparatus for blood sample collection with the simplified safety device according to the invention;
Figure 2 is an elevational view of the safety device according to the invention;
Figure 3 is an axial sectional view of the safety device taken along the sectional plane III-III of Figure 2;
Figure 4 is an elevational view of the sleeve of the blood collection apparatus according to the invention;
Figure 5 is an axial sectional view of the sleeve taken along the sectional plane V-V of Figure 4;
Figure 6 is an axial sectional view of the blood sample collection apparatus according to the invention assembled and in the working position, in which the needle assembly is shown on view;
Figure 7 is an enlarged elevational view of the apparatus for blood sample collection in which the kinematic mechanism of the safety device for passing from the working position to the safe position is shown with dashed lines;
Figure 8 is an axial sectional view of the blood sample collection apparatus in the safe position;
Figure 9 is an elevational view of the blood sample collection apparatus in the safe position.

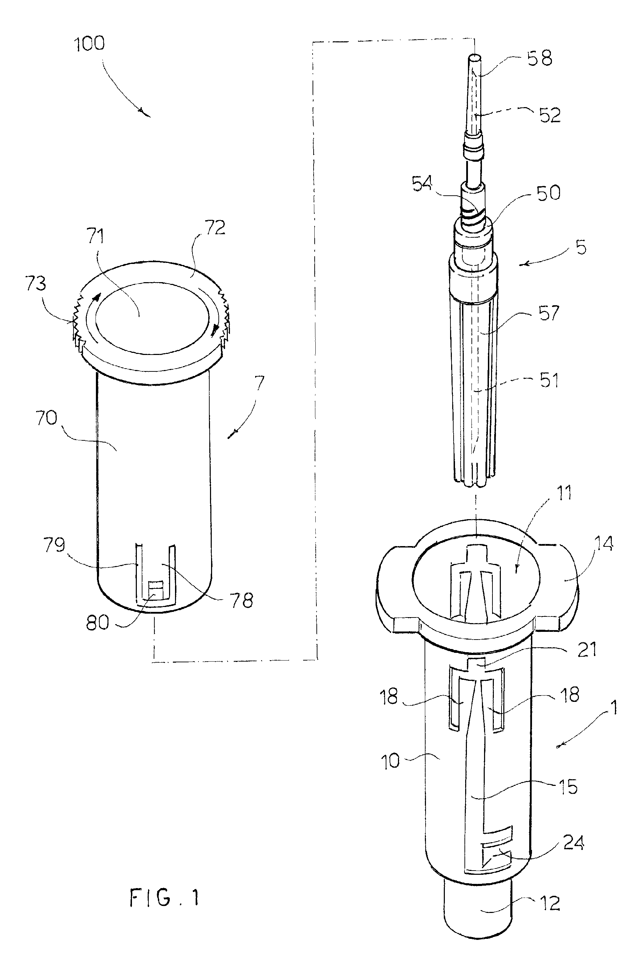

An apparatus for collection of blood samples denoted as a whole by reference numeral 100 will be described with the aid of the figures. As shown in Figure 1, the blood collection apparatus 100 comprises a body or sleeve 1, a needle assembly 5 and a safety device 7.

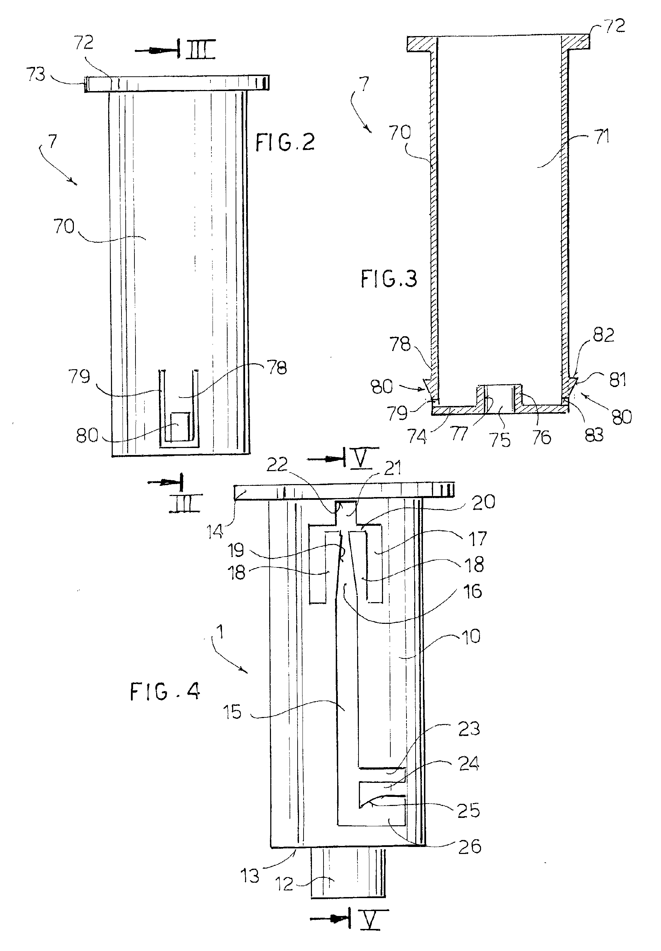

As also shown in Figures 2 and 3, the safety device 7 comprises a substantially cylindrical body 70 hollow on the inside, defining an inner chamber 71 open at the front and rear. The inner chamber 71 of the body of the safety device 70 is sufficiently large to receive a vacutainer-type test tube for blood collection. At the rear end the safety device 7 has a radial flange 72 having a knurled side surface 73 in order to facilitate rotation thereof by the operator.

The body of the safety device 7 has at its front end a radial part 74 which has an axial through hole 75. Around the hole 75 a cylindrical tang 76 protruding axially inward extends around the hole 75. The tang 76 has an internal thread 77.

Two longitudinal tongues 78 disposed in diametrically opposite positions are provided in the side wall of the body 70, near the front end. Each flexible longitudinal tongue 78 is defined by a substantially U-shaped groove 79. In this manner the tongues 78 can bend radially inward and outward with respect to the axis of the safety device 7.

Each tongue 78 has an outward facing protrusion 80. Each protrusion 80 is defined by a tapered lateral surface 81, a rear radial abutment surface 82 and a front radial abutment surface 83.

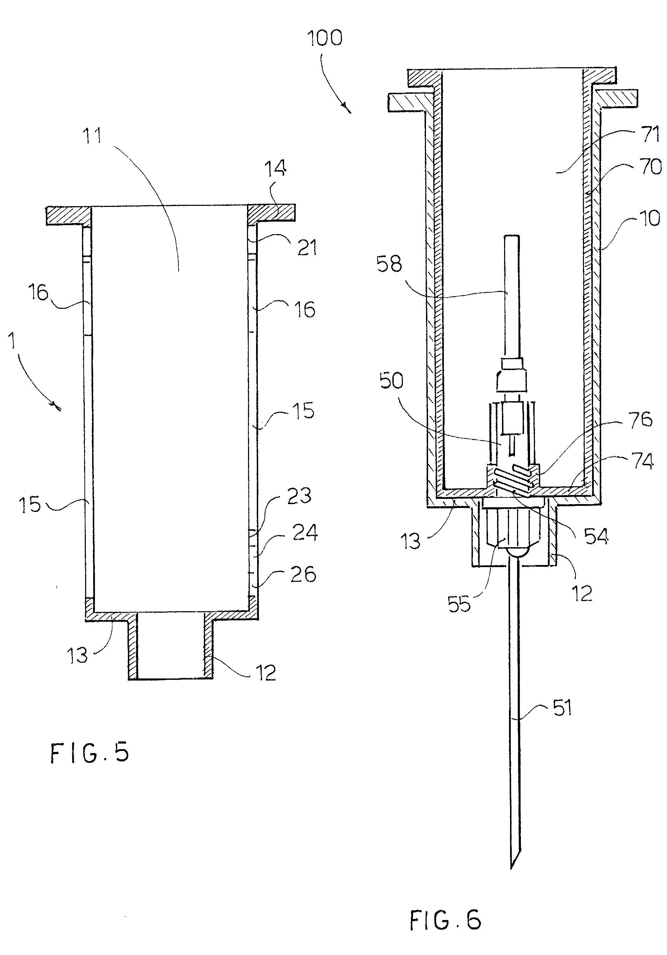

As also shown in Figures 4 and 5, the sleeve 1 comprises a substantially cylindrical body 10, hollow on the inside, defining a cylindrical chamber 11. The inside diameter of the body 10 of the sleeve 1 is equal to or slightly greater than the outside diameter of the body 70 of the safety device 7, so that the safety device 7 can be received inside the chamber 11 of the sleeve.

The sleeve 1 has in its front part an axial head 12 in the form of a cylindrical tang, smaller in diameter than the body 10 and outwardly open. In this manner a shoulder 13 is defined between the body 10 of the sleeve 1 and the head 12. In the rear part the body 10 of the sleeve 1 is outwardly open and has a radially protruding flange 14.

The body 10 of the sleeve 1 has two longitudinal guide grooves 15 disposed in diametrically opposite positions and extending for almost the entire length of the body 10. The grooves 15 can be through slots or can be grooves formed in the inner surface of the body 10 of the sleeve 1.

Each longitudinal slot 15 has a narrowing 16 at the rear defined by the opposite facing tapered surfaces 19 of the two flexible longitudinal tongues 18. The flexible longitudinal tongues 18 are defined by a slot 17 shaped like an upturned U and by the narrowing 16 disposed centrally to the U.

The longitudinal tongues 18 have a rear abutment surface 20. Each tongue 18 can bend in the upturned U-shaped slot 17 so as to widen the narrowing 16 in the longitudinal slot 15.

Rearward of the narrowing 16, the longitudinal guide slot 15 ends in a rear stop seat 21 defined by a rear end abutment surface 22.

A C-shaped slot 23 is provided in the front end of each longitudinal guide slot 15. The C-shaped slot comprises two circumferential slots substantially parallel to each other so as to define between them a flexible circumferential tongue 24. In this manner the flexible circumferential tongue 24 can bend forward and backward inside the C-shaped slot 23.

The flexible circumferential tongue 24 has a substantially tapered front abutment surface 25, acting as a retaining surface. Forward of the circumferential tongue 24, the C-shaped slot 23 defines a front end stop seat 26 communicating with the longitudinal groove 15 and disposed at a right angle thereto.

As shown in Figure 1, the needle assembly 5 comprises a substantially cylindrical needle-carrier 50, hollow on the inside, and carrying axially at its two opposite ends a first external needle 51 and a second internal needle 52, smaller in length than the external needle. The external needle 51 is designed to be positioned outside the sleeve 1 for insertion in the patient's vein for collection of a blood sample and the internal needle 52 is designed to be positioned in the chamber 71 of the safety device 7 to engage with a vacutainer-type test tube. Since the inside of the needle carrier 50 is hollow, the inner channels of the external needle 51 and of the internal needle 52 are communicating.

The two needles 51 and 52 are covered by respective needle caps 57, 58 which removably engage on the ends of the needle-carrier 50. An external thread 54 able to engage in the internal thread 77 of the axial tang 76 of the safety device 7, as shown in figure 6, is formed in the outer side surface of the needle-carrier. On the needle-carrier 50, between the external thread 54 and the end that receives the external needle 51 a bushing 55 is provided able to be gripped by the operator to screw the needle assembly 5 into the safety device 7.

Assembly and operation of the blood sample collection device 100 will now be described with reference to Figures 6-9.

Initially, the needle assembly 5 is screwed into the safety device 7, then the safety device 7 with the needle assembly 5 is inserted from the rear inside the chamber 11 of the sleeve 1. The protrusions 80 of the tongues 78 of the safety device slide inside the body 10 of the sleeve, causing the tongues 78 to bend inward until the protrusions 80 snap into the front end stop seat 26 of the C-shaped slot 23.

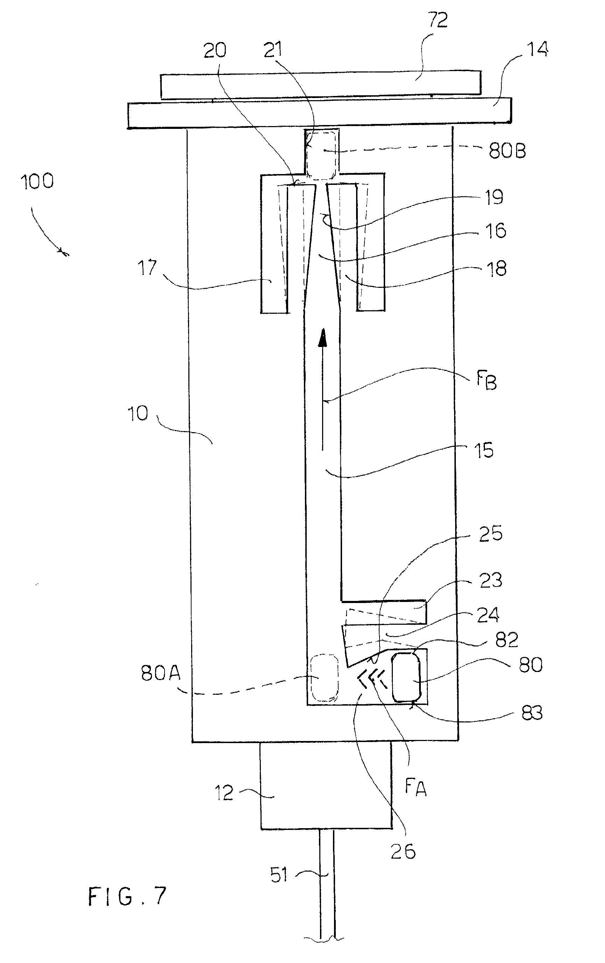

In this situation, as shown in Figure 6, the radial part 74 of the safety device 7 abuts against the shoulder 13 of the sleeve and, as shown in Figure 7, the rear abutment surface 82 of the protrusions 80 of the longitudinal tongues 78 of the safety device abuts against the front abutment surface 25 of the circumferential tongues 24 of the sleeve 1. Consequently the safety device 7 is retained inside the sleeve 1, avoiding any axial movement of the safety device with respect to the sleeve.

In this situation (Figure 6) the bushing 55 of the needle-carrier 5 is situated inside the head 12 of the sleeve, the external needle 51 protrudes axially out of the head of the sleeve and the internal needle 52 is disposed axially inside the chamber 71 of the safety device.

In this situation the blood sample collection apparatus 100 is ready for use. The caps 57 and 58 are then removed, the external needle 51 is inserted into the patient's vein and a vacutainer-type test tube is inserted into the chamber 71 of the safety device to be engaged by the internal needle 52. Consequently the vacuum maintained inside the vacutainer tube causes the blood to be drawn. The blood from the patient's vein is thus drawn through the external needle 51, the needle-carrier 50 and the internal needle 52 into the vacutainer test tube without manual intervention by the operator.

When the vacutainer tube is full of blood, the operator removes the test tube from the chamber 71 of the safety device 7 and seals it to send it to the centre for analysis or blood collection.

When sampling has been completed, that is, when the required number of test tubes has been filled, the operator rotates the safety device 7 (clockwise with reference to Figure 7), by means of the flange 72. Consequently the protrusions 80 of the tongues 78 of the safety device 7 turn in the direction of the arrow F_{Λ} inside the front stop seat 26. During this rotation, the rear abutment surface 82 of the protrusion 80 slides on the tapered front surface 25 of the circumferential tongue 24 of the sleeve causing bending of the circumferential tongue 24, as shown by the dashed line in Figure 7, until the protrusion 80 reaches the limit of rotation, at the longitudinal groove 15 of the sleeve, in the position indicated by the dashed line with reference numeral 80A.

At this point further clockwise rotation of the safety device is prevented. Thus the safety device is pulled axially upward, in the direction of extraction of the sleeve. Consequently the protrusion 80 of the safety device which was in position 80A slides in the longitudinal groove 15 of the sleeve in the direction of arrow F_{B}.

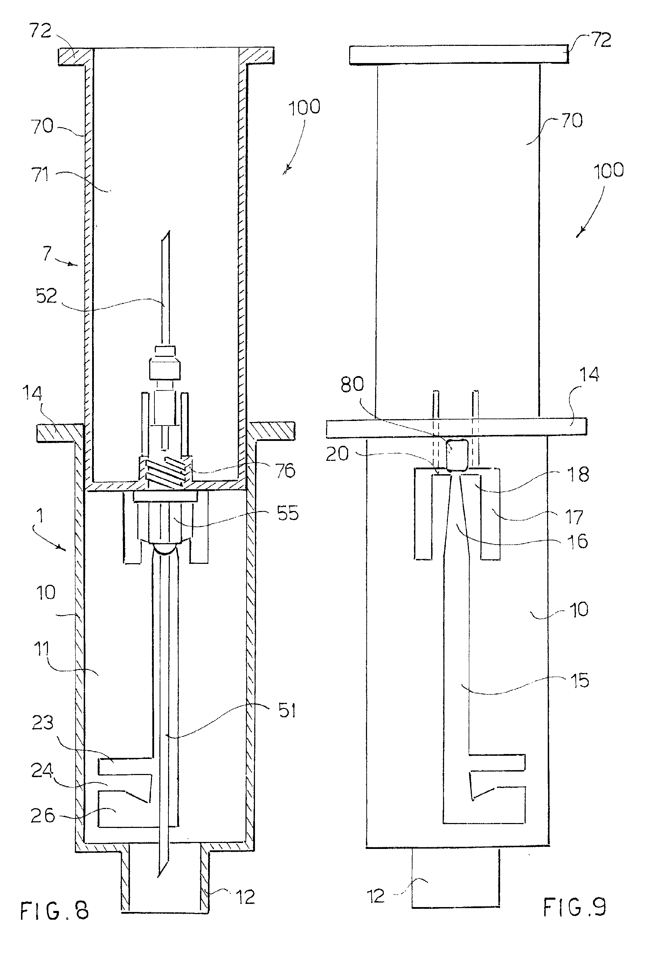

When the protrusion 80 of the safety device passes through the narrowing 16 of the longitudinal groove 15 of the sleeve, it slides on the tapered surfaces 19 of the longitudinal tongues 18 of the sleeve and causes bending of the tongues 18 which are divaricated, widening the narrowing 16 so as to allow the passage of the protrusion 80 of the longitudinal tongue 78 of the safety device which passes through the narrowing 16 to stop inside the end stop seat 21 in the position illustrated with the dashed line and denoted with reference numeral 80B. In this situation of safety, as shown in Figure 8, the external needle 51 is protected inside the chamber 11 of the sleeve 1 and the internal needle 52 is protected inside the chamber 71 of the safety device 7.

As shown in Figure 9, once the protrusion 80 of the longitudinal tongue 78 of the safety device has passed the rear end 20 of the longitudinal tongues 18 of the sleeve, the longitudinal tongues 18 of the sleeve return to the initial position, again forming the narrowing 16, and the rear abutment surface 20 of the longitudinal tongues 18 of the sleeve abuts against the front abutment surface 83 of the protrusion 80 of the longitudinal tongue 78 of the safety device.

In this situation of safety, therefore, any axial movement of the safety device 7 with respect to the sleeve 1 is prevented. In fact the rear surface 82 of the protrusion 80 of the longitudinal tongue of the safety device is in abutment against the stopping surface 22 of the rear stop seat 21 and the front surface 83 of the protrusion 80 of the longitudinal tongue of the safety device is in abutment against the rear abutment surface 20 of the longitudinal tongues 18 of the sleeve.

Numerous variations and modifications of detail within the reach of a person skilled in the art can be made to the present invention, without departing from the scope of the invention, set forth in the appended claims.

## Claims

1. An apparatus (100) for collection of blood samples comprising:
- a sleeve (1) having a substantially cylindrical body (10), hollow on the inside, defining an inner chamber (11) open at the front and rear, and
- a needle assembly (5) comprising a needle-carrier (50) which carries at its opposite ends a first external needle (51) designed to be inserted into the patient's vein and a second internal needle (52) designed to engage in a vacutainer-type test tube,
**characterized in that** it further comprises
- a safety device (7) having a substantially cylindrical body (70), hollow on the inside, defining an inner chamber (71) able to receive a vacutainer-type test tube, said safety device being able to support said needle assembly (5) so that said second internal needle (52) is disposed axially inside the chamber (71) of said safety device and said safety device (7) being movably mounted inside said chamber (11) of the sleeve (1) to pass from a working position in which said first external needle (51) of the needle assembly protrudes axially outward from said sleeve (1) to a safe position in which said first external needle (51) is protected inside the chamber (11) of said sleeve (1) and said second internal needle (52) is protected inside the chamber (71) of said safety device (7).

2. An apparatus for collection of blood according to claim 1, **characterized in that** provided inside said body (10) of said sleeve (1) are first engagement means (24) and second engagement means (18) cooperating with mutual engagement means (78) provided in the body (70) of said safety device (7) to block said safety device respectively in said working position and in said safe position.

3. An apparatus for collection of blood samples according to claim 2, **characterized in that** said first engagement means of the sleeve (1) are flexible tongues (24) circumferentially disposed on the body (10) of the sleeve and said mutual engagement means of the safety device (7) are flexible tongues (78) disposed longitudinally on the body (70) of the safety device and having a protrusion (80) protruding outward to abut against an abutment surface (25) of said circumferential tongue (24) of the sleeve so as to avoid axial movement of the safety device (7) with respect to the sleeve (1).

4. An apparatus for collection of blood samples according to claim 3, **characterized in that** the abutment surface (26) of said circumferential tongues (24) of the sleeve is substantially tapered.

5. An apparatus for collection of blood samples according to claim 3 or 4, **characterized in that** each circumferential tongue (24) of the sleeve is defined by a substantially C-shaped slot (23) formed in the front side wall of the body (10) of said sleeve and each longitudinal tongue (78) of the safety device is defined by a substantially U-shaped slot (79), formed in the front side wall of the body (70) of said safety device.

6. An apparatus for collection of blood samples according to any one of claims 3 to 5, **characterized in that** said circumferential tongues (24) of the sleeve (1) are disposed in diametrically opposite positions on the body (10) of the sleeve and said longitudinal tongues (78) of the safety device (7) are disposed in diametrically opposite positions on the body (70) of the safety device.

7. An apparatus for collection of blood samples according to claim 5 or 6, **characterized in that** each C-shaped slot (23) of the sleeve is connected to a guide slot or groove (15) formed longitudinally along the body (10) of the sleeve and ending in a seat (21) in the rear part of the body of the sleeve so that said protrusion (80) of the longitudinal tongue (78) of the safety device can travel along said guide groove (15) until it stops in said seat (21) when the safety device is in the safe position.

8. An apparatus for collection of blood samples according to claim 5 or 6, **characterized in that** said longitudinal guide grooves (15) are disposed in the body (10) of said sleeve in diametrically opposite positions.

9. An apparatus for collection of blood samples according to claim 7 or 8, **characterized in that** upstream of said stop seat (21) said longitudinal groove (15) of the sleeve has a narrowing (16), defined by two flexible longitudinal tongues (18) having a rear abutment surface (20) able to abut against the front surface of said protrusion (80) of the longitudinal tongues (78) of the safety device, when said protrusion (80) is in said stop seat (20) of the sleeve to avoid axial movements of the safety device when it is in the safe position.

10. An apparatus for collection of blood samples according to any one of the preceding claims, **characterized in that** said safety device (70) has a radial front part (74) with an inwardly protruding tang (76) having an internal thread (77) able to engage with an external thread (54) provided on the body (50) of said needle assembly.
